# EUROPÄISCHE PATENTANMELDUNG

(11) **EP 4 275 661 A1**
(43) Veröffentlichungstag der Anmeldung: **15.11.2023**
(21) Anmeldenummer: 23173131.6
(22) Anmeldetag: 12.05.2023
(51) Int. Cl.: A61F 5/058, A61L 15/08

(54) **SCHIENVORRICHTUNG UND EINE SOLCHE UMFASSENDES SCHIENSYSTEM**

(30) Priorität: 13.05.2022 DE 202022102644 U
(71) Anmelder: X-CEN-TEK GmbH & Co. KG, 26203 Wardenburg (DE)
(72) Erfinder: HARMS, Andreas, 26160 Bad Zwischenahn (DE)
(74) Vertreter: Meyer, Thorsten

(57) **Zusammenfassung**

Die Erfindung betrifft eine Schienvorrichtung (01), mit mindestens einen dünnwandigen Streifen (02) als versteifende Komponente, wobei zumindest einseitig des dünnwandigen Streifens (02) ein erstes Fasermaterial (03) angeordnet ist, und einem Band (07) eines zweiten Fasermaterials, wobei das eine und das andere Fasermaterial zusammen als Klettverschluss wirken.

## Beschreibung

Die Erfindung betrifft eine Schienvorrichtung gemäß dem Oberbegriff des Anspruchs 1. Darüber hinaus betrifft die Erfindung ein Schiensystem umfassend eine solche Schienvorrichtung.

Als so genannte Splint-Schienen erstmals bekannt gewordene Traktionsschienen werden am häufigsten zur Behandlung schwerer Knochenmittenfrakturen des Femurs, aber auch von Frakturen anderer Gliedmaßen eingesetzt. Der Femur ist der längste und stärkste Knochen im menschlichen Körper. Die umliegenden Muskeln sind ebenfalls sehr stark. Wenn der Femur bricht, können die umliegenden Muskeln krampfen und dazu führen, dass die Knochenenden aneinander vorbeigezogen werden, was zu immensen Schmerzen, internen oder äußerlichen Blutungen sowie Muskel- und Nervenschäden führt.

Eine Traktionsschiene wirkt dem entgegen, indem die gebrochene Gliedmaße geschient und zugleich eine Zugkraft auf die Fraktur aufgebracht wird.

Die Traktionsschiene umfasst hierzu eine Stange, die parallel zum gebrochenen Bein angelegt gegen das Becken abgestützt wird, und durch Umwickeln mit Verbandsmaterial schienend auf den Femur einwirkt. Am distalen Ende der Stange ist eine Zugvorrichtung angeordnet, die den Fuß des gebrochenen Beins weg vom Becken zieht.

Bei Knochenbrüchen weniger bemuskelter Gliedmaßen ist die Traktionsschiene überdimensioniert. Auch das Aufbringen einer Zugkraft ist hier nicht zwingend notwendig. Ferner ist das Packmaß von Traktionsschienen nachteilig für das Mitführen in der Notfallmedizin zur Erstversorgung an einem Unfallort. Ein zusätzlicher Nachteil ergibt sich durch die komplizierte, zeitaufwändige Anwendung der Traktionsschiene.

Ebenfalls bekannt ist eine Schienvorrichtung zur Ruhigstellung von Knochenbrüchen und anderen Verletzungen. Die Schienvorrichtung besteht aus einem mit einem Mantel beispielsweise aus einem Schaummaterial, wie etwa einem Polyethylenschaum überzogen Kern aus dünnwandigem Aluminium. Die Schienvorrichtung kann zu einer Rolle aufgerollt kompakt gelagert und mitgeführt werden. Abgerollt liegt sie als zunächst rechteckiger, flacher Streifen vor. Eine Gliedmaße lässt sich mit der Schienvorrichtung einfach schienen, indem der Streifen entlang der Gliedmaße zu einer Rinne mit den Kern aus Aluminiumblech versteifendem U-förmigem Querschnitt verformt und mittels Verbandsmaterial an der Gliedmaße fixiert wird.

Nachteilig an der Schienvorrichtung verbleibt deren Anwendung, die des Rückgriffs auf zusätzlich mitzuführende Gegenstände und Verbandsmaterialien bedarf.

Eine Aufgabe der Erfindung ist eine Schienvorrichtung zu schaffen, welche kompakt mitgeführt einfach und schnell universell anwendbar ist. Darüber hinaus ist es eine Aufgabe der Erfindung, ein eine solche Schienvorrichtung umfassendes Schiensystem mit dessen Vorteilen zu entwickeln.

Die Aufgabe wird durch die Merkmale des unabhängigen Anspruchs gelöst. Vorteilhafte Ausführungsformen sind in den Ansprüchen, den Zeichnungen sowie in der nachfolgenden Beschreibung, einschließlich der zu den Zeichnungen zugehörigen, wiedergegeben.

Ein erster Gegenstand der Erfindung betrifft demnach eine als ein Schienmittel von Frakturen und anderen Verletzungen verwendbare Schienvorrichtung. Die Schienvorrichtung kann auch als Splint-Schiene bezeichnet werden. Diese umfasst einen dünnwandigen Streifen als versteifende Komponente. Beispielsweise eingnet sich hierzu ein Aluminiumblech. Alternativ oder zusätzlich kann eine dünne Polymerplatte die versteifende Komponente ganz oder teilweise bilden.

Zumindest einseitig des dünnwandigen Streifens ist ein erstes Fasermaterial angeordnet. Die mit dem ersten Fasermaterial versehene Seite der Splint-Schiene wird auch als Flauschseite bezeichnet.

Darüber hinaus umfasst die Schienvorrichtung mindestens ein Band eines zweiten Fasermaterials.

Das eine Fasermaterial hat flexible Widerhäkchen, das andere Schlaufen.

Das eine und das andere Fasermaterial wirken zusammen als Klettverschluss.

Ein zweiter Gegenstand der Erfindung betrifft ein System einer als ein Schienmittel von Frakturen und anderen Verletzungen verwendbaren, zuvor beschriebenen Schienvorrichtung, sowie eines hinzugefügten, das zweite Fasermaterial umfassenden Klettbands, welches die Schienvorrichtung an dem Patienten durch Wechselwirkung beiderseits der Flauschseite und dem Klettband fixiert.

Die Schienvorrichtung kann einzelne oder eine Kombination der zuvor und/oder nachfolgend in Verbindung mit dem System beschriebene Merkmale aufweisen, ebenso wie das System einzelne oder eine Kombination mehrerer zuvor und/oder nachfolgend in Verbindung mit der Schienvorrichtung beschriebene Merkmale aufweisen und/oder verwirklichen kann.

Die Schienvorrichtung und/oder das System kann alternativ oder zusätzlich einzelne oder eine Kombination mehrerer einleitend in Verbindung mit dem Stand der Technik und/oder in der nachfolgenden Beschreibung zu den in den Zeichnungen dargestellten Ausführungsbeispielen beschriebene Merkmale aufweisen.

Zusätzliche, über die vollständige Lösung der gestellten Aufgabe und/oder über die voran zu den einzelnen Merkmalen genannten Vorteile hinausgehende Vorteile gegenüber dem Stand der Technik sind nachfolgend aufgeführt.

Die Erfindung wird nachfolgend anhand von in der Zeichnung dargestellter Ausführungsbeispiele näher erläutert. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind. Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die Erfindung ausgestaltet sein kann und stellen keine abschließende Begrenzung dar. Es zeigen in schematischer Darstellung:
- Fig. 1: eine Schienvorrichtung 01 und ein diese umfassendes System 10 in einem Ausgangszustand in einer Draufsicht.
- Fig. 2: die Schienvorrichtung 01 und ein diese umfassendes System 10 aus Fig. 1 in einer Seitenansicht.
- Fig. 3: die Schienvorrichtung 01 und ein diese umfassendes System 10 aus Fig. 1 in einer Ansicht von unten.
- Fig. 4: die Schienvorrichtung 01 und ein diese umfassendes System 10 in einem Anwendungszustand in einer perspektivischen Ansicht.
- Fig. 5: ein erstes Ausführungsbeispiel eines mehrlagigen Aufbaus einer Schienvorrichtung 01 in perspektivischer Explosionsdarstellung.
- Fig. 6: ein zweites Ausführungsbeispiel eines mehrlagigen Aufbaus einer Schienvorrichtung 01 in perspektivischer Explosionsdarstellung.

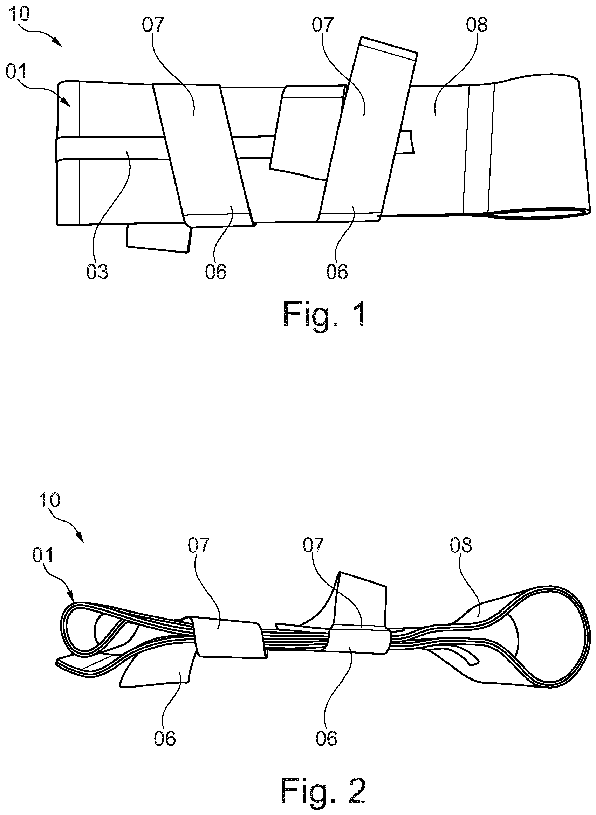

Eine in Fig. 1, Fig. 2, Fig. 3, Fig. 4, Fig. 5, Fig. 6 ganz oder in Teilen dargestellte, als ein Schienmittel von Frakturen und anderen Verletzungen verwendbare Schienvorrichtung 01 umfasst:
- mindestens einen dünnwandigen Streifen 02 als versteifende Komponente, wobei zumindest einseitig des dünnwandigen Streifens ein erstes Fasermaterial 03 angeordnet ist.
- mindestens ein Band 07 eines zweiten Fasermaterials 06, wobei das eine und das andere Fasermaterial 03, 06 zusammen als Klettverschluss wirken.

Die mit dem ersten Fasermaterial 03 versehene Seite der Splint-Schiene wird auch als Flauschseite bezeichnet.

Um zusammen als Klettverschluss zu wirken, weist das eine Fasermaterial 03, 06 vorteilhaft flexible Widerhäkchen auf, und das andere Fasermaterial 06, 03 weist vorteilhaft Schlaufen auf.

Die Schienvorrichtung 01 kann auch als Splint-Schiene bezeichnet werden.

Beispielsweise eingnet sich als versteifende Komponente ein Aluminiumblech. Alternativ oder zusätzlich kann eine dünne Polymerplatte die versteifende Komponente ganz oder teilweise bilden.

Der dünnwandige Streifen 02 kann aus einem gegenüber innerhalb der durch ihn eingenommenen Fläche wirkenden Zug- und Druckkräften formstabilen, insbesondere steifen Material bestehen. Gegenüber senkrecht zu der von ihm eingenommenen Fläche ist das Material des dünnwandigen Streifens 02 vorteilhaft verformbar, beispielsweise biegeelastisch oder plastisch verformbar.

Besonders bevorzugt besteht der dünnwandige Streifen 02 aus einem Aluminiumblech.

Die Schienvorrichtung 01 kann einstückig ausgebildet sein.

Das eine oder die mehreren Bänder 07 sind demnach mit dem mit dem ersten Fasermaterial 03 versehenen dünnwandigen Streifen 02 verbunden. Die Verbindung kann untrennbar sein, wobei eine Trennung der Bänder 07 von dem mit dem mit dem ersten Fasermaterial 03 versehenen dünnwandigen Streifen 02 nur durch mechanische Zerstörung, beispielsweise Durchtrennen mittels einer Schere, beispielsweise der Bänder 07 an der Verbindungsstelle zum mit dem ersten Fasermaterial 03 versehenen dünnwandigen Streifen 02 möglich ist. Alternativ kann die Verbindung lösbar sein, wobei eine Wiederherstellung der Verbindung möglich ist. Beispielsweise kann das oder können die Bänder 07 mittels der Klettwirkung ihres zweiten Fasermaterials 06 mit dem mit dem ersten Fasermaterial 03 versehenen dünnwandigen Streifen 02 verbunden sein.

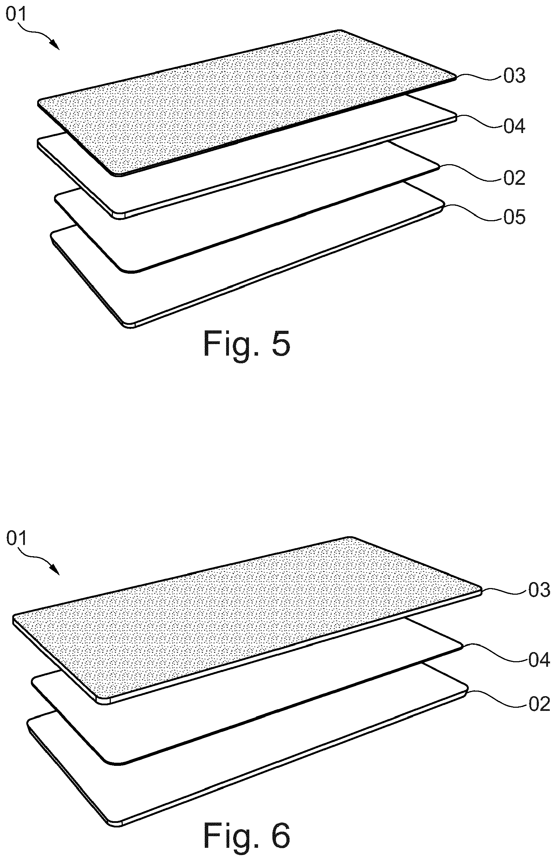

Die Schienvorrichtung 01 ist bevorzugt mehrlagig aufgebaut, wie in Fig. 5 und Fig. 6 dargestellt. Die Schienvorrichtung 01 besteht hiernach zumindest aus dem dünnwandigen Streifen 02, beispielsweise als Kern, und einer das erste Fasermaterial 03 umfassenden Lage als Deckschicht.

Das erste Fasermaterial 03 kann vollflächig eine Ober- und/oder Unterseite 08 der Schienvorrichtung bilden. Alternativ kann es einen Abschnitt auf einer Ober- oder Unterseite 08 der Schienvorrichtung 01 einnehmen, wie in Fig. 1, Fig. 2, Fig. 3 dargestellt.

Das erste Fasermaterial 03 kann eine beispielsweise durch die Ober- oder Unterseite 08 gebildete oberste und/oder unterste Lage der Schienvorrichtung ganz oder teilweise bilden oder umfassen. Es kann alternativ oder zusätzlich von einer beispielsweise durch die Ober- oder Unterseite 08 gebildete obersten und/oder untersten Lage der Scheinvorrichtung 01 gebildet oder umfasst sein.

Bevorzugt bildet das zweite Fasermaterial 06 das Band 07, umfasst dieses oder wird ganz oder teilweise von dem Band 07 gebildet oder umfasst.

Vorteilhaft sind entlang der Längserstreckung des Streifens 02 mehrere quer zu dieser angeordnete Bänder 07 vorgesehen. Entlang der Längserstreckung des Streifens 02 sind hiernach mehrere Bänder 07 quer zu dieser angeordnet.

Eine oder mehrere zusätzliche Polsterlagen 04, 05 und/oder Schutzschichten können zwischen dem ersten Fasermaterial 03 und dem dünnwandigen Streifen 02 und/oder auf der dem ersten Fasermaterial 03 abgewandten Seite des dünnwandigen Streifens 02 angeordnet sein.

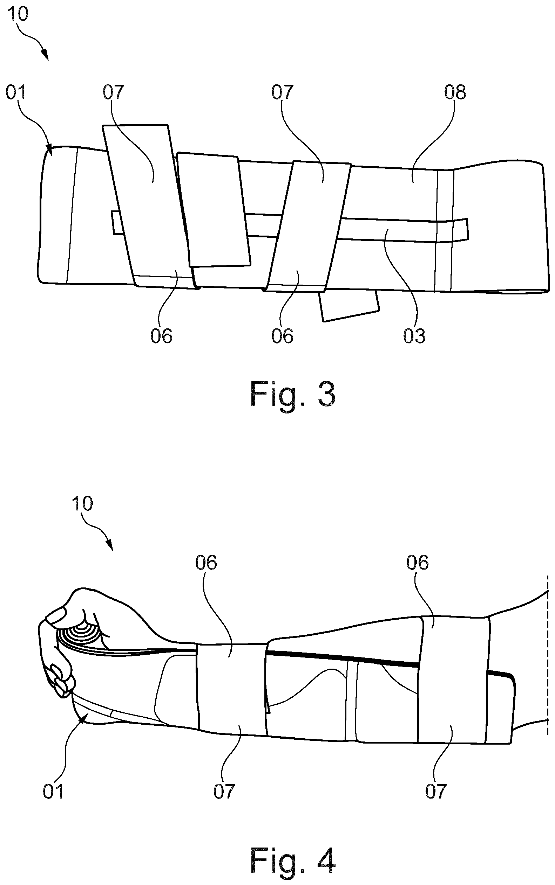

Es ist ersichtlich, dass die Erfindung beispielsweise verwirklicht sein kann durch eine Splint-Schiene zur Fixierung von Körperteilen eines Patienten. Die Schiene ist verformbar ausgestaltet, um von einem Flächenmaterial (Fig. 1, Fig. 2, Fig. 3) auf ein an das Körperteil angepasstes Material (Fig. 4) versetzt werden zu können. Die Schiene ist mit einer ersten Polsterungsschicht 05, einem darauf befestigten, dünnwandigen Streifen 02 als versteifende Komponente, insbesondere einer Aluminium- und/oder Metalllage, wie etwa einem Aluminiumblech, beispielsweise als Kern, und einer zweiten Polsterung 04 auf dem dünnwandigen Streifen 02 versehen.

Die Spilint-Schiene zeichnet sich dadurch aus, dass auf der zweiten Polsterung 04 ein flauschiges Textil, ähnlich eines Flauscherbands einer Klettverbindung, als das erste Fasermaterial 03 aufgebracht ist, wobei die zweite Polsterung 04 alternativ oder zusätzlich ein solches Textil umfasst, oder von einem derartigen Textil umfasst ist.

Die Splint-Schiene umfasst vorteilhaft ein Band 07 auf das das zweite Fasermaterial 06 aufgebracht ist. Das Band 07 kann alternativ oder zusätzlich ein solches Fasermaterial 06 umfassen, oder von einem derartigen Fasermaterial 06 umfasst sein.

Das Band 07 kann beispielsweise mittels einer Schere in Abschnitte geeigneter und/oder benötigter Länge zerteilt werden.

Alternativ kann das Band 07 bereits in mehrere Abschnitte unterteilt und/oder an mehreren Stellen der Splint-Schiene einseitig befestigt angeordnet sein. Mit dem oder mit den jeweils freien distalen Enden des oder der mehreren Bänder 07 kann die Splint-Schiene an das Körperteil angepasst und durch Verhaken mit dem ersten Fasermaterial 03 zugleich an dem Körperteil fixiert werden.

Wichtig ist hervorzuheben, dass die Erfindung verwirklicht sein kann durch ein in Fig. 1, Fig. 2, Fig. 3, Fig. 4, Fig. 5, Fig. 6 ganz oder in Teilen dargestelltes System 10 einer zuvor beschriebenen, als ein Schienmittel von Frakturen und anderen Verletzungen verwendbaren Schienvorrichtung 01 oder einer Splint Schiene, sowie eines hinzugefügten, das zweite Fasermaterial 06 umfassenden Klettbands 07. Mittels des Klettbands 07 wird die zunächst in ihrer Ausgangsgestalt vorliegende und manuell an ein zustützendes Körperteil eines Patienten angepasste Splint-Schiene in ihrer angepassten Gestalt an dem Patienten durch Wechselwirkung beiderseits der Flauschseite und dem Klettband 07 befestigt.

Bei dem System 10 wird die Schienvorrichtung 01 an dem Patienten durch Wechselwirkung beiderseits der Flauschseite und dem Klettband 07 fixiert.

Wichtig ist hervotzuheben, dass der der Erfindung zu Grunde liegende Gedanke in einer beispielsweise durch eine flauschige Oberseite gebildeten Flauschseite einer auch als Splint-Schiene bezeichenbaren Schienvorrichtung 01 liegt. Diese durch ein erstes Fasermaterial 03 gebildete, von einem solchen umfasste oder ein erstes Fasermaterial 03 bildende oder umfassende Flauschseite dient dem Anbringen von Klettstreifen. Diese sollen einem durch das System aus Schienvorrichtung 01 und Band 07 gebildeten Produkt beiliegen und benutzt werden, um die Splint-Schiene an die zu stabilisierende Gliedmaße zu fixieren. Es muss kein externes Verbandmaterial mehr verwendet werden und die Versorgung des Patienten ist schneller durchführbar.

Als eine rudimentäre Umsetzung der Idee kann ein schmales Stück des ersten Fasermaterials 03 als Flauschseite auf eine eine Ober- und/oder Unterseite 08 der Splint-Schiene bildende Seite geklebt werden, um die Splint-Schiene mittels zweier als Klettstreifen ausgeführter Bänder 07 an beispielsweise einem Arm zu fixieren. Die Funktionsweise ist eindeutig gegeben.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Ansprüchen beinhaltet, auch wenn dieses Merkmal oder diese Kombination selbst nicht explizit in den Ansprüchen oder Ausführungsbeispielen angegeben ist.

Die Erfindung ist insbesondere im Bereich der Herstellung von medizintechnischen Gegenständen, insbesondere solchen zur medizinischen Nothilfe und/oder Erstversorgung gewerblich anwendbar.

Die Erfindung wurde unter Bezugnahme auf bevorzugte Ausführungsformen beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 01: Schienvorrichtung
- 02: Streifen
- 03: erstes Fasermaterial
- 04: Polsterlage
- 05: Polsterlage
- 06: zweites Fasermaterial
- 07: Band
- 08: Ober- und/oder Unterseite

- 10: System

## Patentansprüche

1. Schienvorrichtung (01), umfassend:
- mindestens einen dünnwandigen Streifen (02) als versteifende Komponente, wobei zumindest einseitig des dünnwandigen Streifens (02) ein erstes Fasermaterial (03) angeordnet ist, und
- ein Band (07) eines zweiten Fasermaterials, wobei das eine und das andere Fasermaterial zusammen als Klettverschluss wirken.

2. Schienvorrichtung nach Anspruch 1, wobei die Schienvorrichtung (01) einstückig ausgebildet ist.

3. Schienvorrichtung nach Anspruch 1 oder 2, wobei die Schienvorrichtung (01) mehrlagig ist, bestehend zumindest aus dem dünnwandigen Streifen (02) und einer das erste Fasermaterial (03) umfassenden Lage als Deckschicht.

4. Schienvorrichtung nach Anspruch 1, 2 oder 3, wobei das erste Fasermaterial (03) vollflächig eine Ober- und/oder Unterseite (08) bildet.

5. Schienvorrichtung nach Anspruch 1, 2 oder 3, wobei das erste Fasermaterial (03) einen Abschnitt auf einer Ober- und/oder Unterseite (08) einnimmt.

6. Schienvorrichtung nach einem der voranstehenden Ansprüche, wobei das erste Fasermaterial (03) eine oberste und/oder unterste Lage (08) der Schienvorrichtung (01) bildet oder umfassst, und/oder von einer obersten und/oder untersten Lage (08) der Scheinvorrichtung (01) gebildet oder umfasst ist.

7. Schienvorrichtung nach einem der voranstehenden Ansprüche, wobei das zweite Fasermaterial das Band (07) bildet, dieses umfasst oder ganz oder teilweise von dem Band (07) gebildet oder umfasst ist.

8. Schienvorrichtung nach einem der voranstehenden Ansprüche, wobei entlang der Längserstreckung des Streifens (02) mehrere Bänder (07) quer angeordnet sind.

9. Schienvorrichtung nach einem der voranstehenden Ansprüche, wobei eine oder mehrere zusätzliche Polsterlagen (04, 05) und/oder Schutzschichten zwischen dem ersten Fasermaterial (03) und dem dünnwandigen Streifen (02) und/oder auf der dem ersten Fasermaterial (03) abgewandten Seite des dünnwandigen Streifens (02) angeordnet sind.

10. System (10) einer als ein Schienmittel von Frakturen und anderen Verletzungen verwendbaren Schienvorrichtung (01) nach einem der voranstehenden Ansprüche, sowie eines hinzugefügten, das zweite Fasermaterial (06) umfassenden Klettbands (07), welches die Schienvorrichtung (01) an einem zu scheinenden Körperteil eines Patienten durch Wechselwirkung beiderseits des ersten Fasermaterials (03) und dem Band (07) fixiert.
